# FASCICULE DE BREVET EUROPEEN

(11) **EP 3 182 935 B1**
(45) Date de publication et mention de la délivrance du brevet: **07.08.2019**
(21) Numéro de dépôt: 15766899.7
(22) Date de dépôt: 07.08.2015
(51) Int. Cl.: A61F 2/42

(54) **IMPLANT D'ARTHRODESE ET INSTRUMENT DE PREHENSION D'UN TEL IMPLANT**
ARTHRODESEIMPLANTAT UND INSTRUMENT ZUM GREIFEN SOLCH EINES IMPLANTATS
ARTHRODESIS IMPLANT AND INSTRUMENT FOR GRIPPING SUCH AN IMPLANT

(30) Priorité: 18.08.2014 FR 1457860
(43) Date de publication de la demande: 28.06.2017
(73) Titulaire: In2Bones, 69130 Ecully (FR)
(72) Inventeur: BOUBLIL, Daniel Edmond, 69006 Lyon (FR); COILLARD-LAVIROTTE, Jean-Yves Paul Albert, 69450 Saint Cyr au Mont d'Or (FR)
(74) Mandataire: Weber, Jean-François
(86) Numéro de dépôt international: PCT/FR2015/052179
(87) Numéro de publication internationale: WO 2016/027025

(56) Documents cités:
- EP-A2- 0 611 557
- WO-A1-2011/110784
- FR-A1- 2 935 601
- US-A1- 2009 210 016

## Description

### DOMAINE TECHNIQUE

L'invention se rapporte au domaine général des implants chirurgicaux d'ostéosynthèse, et en particulier aux implants phalangiens destinés à être mis en place dans le corps d'un patient, au niveau de la main ou du pied de ce dernier, afin de permettre la fusion de deux phalanges.

L'invention concerne plus précisément un implant d'arthrodèse permettant de favoriser la fusion osseuse d'un premier os avec un deuxième os, ledit implant comprenant :
- un corps d'ancrage sensiblement rigide et non déformable, conçu pour assurer l'ancrage de l'implant dans le premier os, le corps d'ancrage s'étendant entre une extrémité de pénétration dans ledit premier os et une extrémité de base opposée, et
- une portion déformable conçue pour assurer l'ancrage de l'implant dans le deuxième os, ladite portion déformable comprenant au moins deux bras d'ancrage qui font saillie du corps d'ancrage à partir de l'extrémité de base de ce dernier, les bras d'ancrage étant séparés l'un de l'autre par un espace libre de manière à pouvoir être rapprochés l'un de l'autre par déformation desdits bras d'ancrage sous l'action du deuxième os.

### TECHNIQUE ANTERIEURE

Afin de traiter certaines pathologies osseuses, et en particulier des phalanges, telles que des déformations inter-phalangiennes, des orteils en griffe ou en marteau, ou encore de l'arthrose, on est parfois amené à effectuer une arthrodèse, c'est-à-dire à provoquer une fusion interosseuse pour fixer un os par rapport à l'autre.

Dans le cas des phalanges par exemple, il est connu de mettre en place un implant intra-médullaire au cours d'une intervention chirurgicale, qui vise à solidariser deux phalanges l'une par rapport à l'autre, afin d'entraîner leur fusion osseuse. En l'espèce, ce type d'implant chirurgical comporte une partie distale destinée à être ancrée dans le canal médullaire de l'une des deux phalanges, et une partie proximale qui peut être ancrée dans le canal médullaire de la deuxième phalange.

De tels implants d'arthrodèse connus sont le plus souvent réalisés de manière à former une pièce d'un seul tenant, ce qui permet d'améliorer leur longévité dans le corps du patient, de manière par exemple à ne pas nécessiter d'être retirés dudit corps. Une telle conception peut parfois permettre en outre de réduire le coût de fabrication.

Néanmoins, l'utilisation d'un seul matériau est susceptible, dans certains cas, de ne pas satisfaire tout à fait à l'anatomie du patient. En effet, chez la plupart des patients, au sein d'un même squelette, certains corps osseux présentent fréquemment des duretés différentes, de sorte que la dureté ou la souplesse d'un implant donné peut, tout en étant particulièrement adaptée à la dureté de l'une des deux phalanges, ne pas être adaptée à la dureté de l'autre phalange. Plus généralement, l'implant peut être inadapté à son environnement de pose, du fait de la grande variabilité de ce dernier.

Une trop grande inadaptation de la dureté du matériau à l'os est susceptible d'être source de douleurs, dans la mesure où l'implant, lorsqu'il est trop rigide ou trop dur compte-tenu des propriétés mécaniques de l'os, peut endommager ledit os à la pose, ou *a posteriori,* notamment dans le cas d'un patient présentant une structure osseuse affaiblie. Cette inadaptation peut être également la cause d'un allongement de la durée de la thérapie, dans la mesure où l'ostéosynthèse, c'est-à-dire la reconstruction osseuse, peut être gênée par la trop grande dureté de l'implant vis-à-vis de l'un des os.

Au contraire, une souplesse trop importante peut causer un déchaussement accidentel de l'implant du canal médullaire, ou encore des infections, en particulier chez un patient âgé. Par ailleurs, un os d'une rigidité trop importante par rapport à l'implant, est susceptible d'user ce dernier, de manière à nuire à sa longévité. Un tel implant présente ainsi le risque, en cas d'usure trop importante, de nécessiter d'être retiré du corps du patient au cours d'une deuxième opération chirurgicale de retrait dudit implant. Bien entendu, cela implique un coût supplémentaire substantiel, une perte de temps, et un risque potentiel pour la santé du patient, notamment dans le cas d'un patient âgé.

Par ailleurs, la pose des implants connus peut parfois s'avérer difficile, notamment dans le cas où l'implant est particulièrement flexible. En effet, si le chirurgien peut recourir à un outil de préhension pour maintenir fermement l'implant au moment de son introduction dans le canal médullaire de l'os, la partie flexible de l'implant qui est introduite peut se déformer de manière inopportune au moment de l'insertion, et rendre cette opération difficile et imprécise.

Ainsi, les implants connus ne semblent pas présenter les caractéristiques suffisantes pour être à la fois faciles à mettre en place, fiables, robustes, et polyvalents pour s'adapter à la morphologie de chaque patient tout en permettant une arthrodèse relativement rapide et indolore.

On connaît du document FR-2 935 601 A1 un implant intramédullaire pour réaliser une arthrodèse entre deux parties d'os, lequel implant est constitué par un corps monobloc de forme générale allongée présentant, à chaque extrémité, des zones d'ancrage avec les parties d'os considérées, l'une des zones étant de section cylindrique, tandis que l'autre zone est de section méplate.

### EXPOSE DE L'INVENTION

Les objets assignés à la présente invention visent en conséquence à remédier aux différents inconvénients énumérés précédemment et à proposer un nouvel implant particulièrement adapté à la physiologie et à la morphologie du patient, et présentant une grande polyvalence d'utilisation et d'adaptation à son environnement de pose.

Un autre objet de l'invention vise à proposer un nouvel implant dont la tenue dans le corps du patient est particulièrement fiable.

Un autre objet de l'invention vise à proposer un nouvel implant particulièrement robuste et résistant.

Un autre objet de l'invention vise à proposer un nouvel implant permettant de limiter la douleur subie par le patient.

Un autre objet de l'invention vise à proposer un nouvel implant permettant de traiter une pathologie osseuse du patient de façon particulièrement efficace, et rapide.

Un autre objet de l'invention vise à proposer un nouvel implant facile à mettre en place dans le corps du patient.

Un autre objet de l'invention vise à proposer un nouvel implant dont la fabrication est relativement aisée.

Un autre objet de l'invention vise à proposer un nouvel implant permettant de réduire le coût de l'intervention chirurgicale de pose de l'implant.

Un autre objet de l'invention vise à proposer un nouvel implant permettant de réduire le risque pour la santé du patient lié à la pose de l'implant et à la thérapie.

Les objets assignés à l'invention sont atteints à l'aide d'un implant d'arthrodèse permettant de favoriser la fusion osseuse d'un premier os avec un deuxième os, ledit implant comprenant :
- un corps d'ancrage sensiblement rigide et non déformable, conçu pour assurer l'ancrage de l'implant dans le premier os, le corps d'ancrage s'étendant entre une extrémité de pénétration dans ledit premier os et une extrémité de base opposée, et
- une portion déformable conçue pour assurer l'ancrage de l'implant dans le deuxième os, ladite portion déformable comprenant au moins deux bras d'ancrage qui font saillie du corps d'ancrage à partir de l'extrémité de base de ce dernier, les bras d'ancrage étant séparés l'un de l'autre par un espace libre de manière à pouvoir être rapprochés l'un de l'autre par déformation desdits bras d'ancrage sous l'action du deuxième os.
et étant caractérisé en ce qu'au moins l'un des bras d'ancrage est pourvu d'une ailette longitudinale de renfort faisant saillie du bras d'ancrage à partir d'un côté latéral de ce dernier.

### DESCRIPTIF SOMMAIRE DES DESSINS

D'autres particularités et avantages de l'invention apparaîtront et ressortiront plus en détails à la lecture de la description faite ci-après, en référence aux dessins annexés, donnés uniquement à titre d'exemple illustratif et non limitatif, dans lesquels :
- La figure 1 illustre, selon une vue en perspective générale, une première variante d'un implant conforme à l'invention, lequel comprend un corps d'ancrage et une portion déformable coaxiaux ;
- La figure 2 représente l'implant de la figure 1 selon une vue de côté ;
- Les figures 3 et 4 illustrent chacune l'implant de la figure 1 respectivement selon une vue axiale face au corps d'ancrage, et selon une vue axiale face à la portion déformable ;
- La figure 5 illustre une deuxième variante d'un implant conforme à l'invention selon une vue en perspective générale, pour lequel le corps d'ancrage et la portion déformable sont inclinés l'un par rapport à l'autre d'environ 10 degrés ;
- Les figures 6 et 7 illustrent l'implant de la figure 5 selon des vues de côté ;
- La figure 8 représente, selon une vue de côté, une troisième variante d'un implant conforme à l'invention pour lequel le corps d'ancrage et la portion déformable sont inclinés l'un par rapport à l'autre d'environ 17 degrés ;
- Les figures 9 et 10 illustrent chacune l'implant de la figures 8 respectivement selon une vue axiale face au corps d'ancrage, et selon une vue axiale face à la portion déformable ;
- Les figures 11 et 12 représentent, selon des vues en perspective, un instrument de préhension, permettant de saisir l'implant des figures 1 à 10 ;
- Les figures 13 et 14 représentent, de façon schématique selon des vues de côté, l'instrument de préhension des figures 11 et 12 associé à l'implant des figures 5 à 10;
- Enfin, les figures 15 et 16 illustrent des instruments d'ouverture d'un canal médullaire d'un os préalablement à l'ancrage de l'implant des figures 1 à 10 dans ledit os.

### MEILLEURE MANIERE DE REALISER L'INVENTION

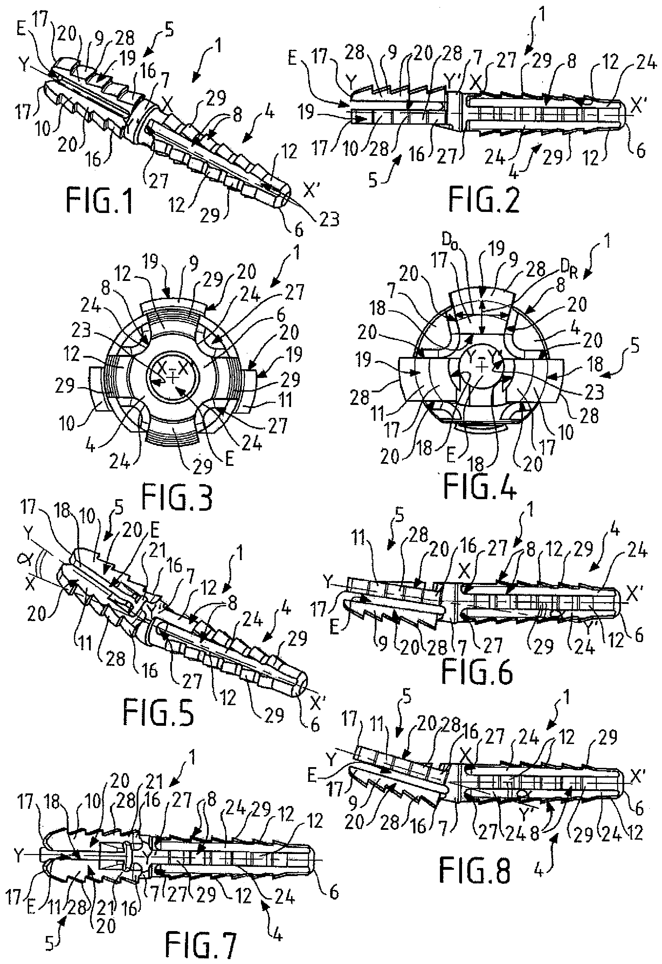

L'invention concerne en tant que tel un implant 1 d'arthrodèse (tel qu'illustré par exemple aux figures 1 à 10), c'est-à-dire un implant 1 chirurgical destiné à être mis en place dans le corps d'un patient, humain ou animal, au niveau de ses os, par exemple au cours d'une opération chirurgicale effectuée sous anesthésie locale ou générale. L'implant 1 de l'invention permet d'effectuer une arthrodèse d'un premier os 2 avec un deuxième os 3, c'est-à-dire qu'il permet de favoriser la fusion osseuse dudit premier os 2 avec ledit deuxième os 3. A l'issue d'une arthrodèse réussie et effectuée à l'aide de l'implant 1, le premier os 2 et le deuxième os 3 se trouvent avantageusement fixés l'un par rapport à l'autre de façon solidaire. La fusion osseuse est préférentiellement effectuée au niveau d'une articulation connectant le premier os 2 au deuxième os 3, de manière à remplacer cette dernière.

Une telle opération chirurgicale peut par exemple permettre, dans le cas où le patient souffre par exemple d'une arthrose interarticulaire, de supprimer la douleur subie par le patient en fusionnant l'articulation atteinte. Alternativement, l'opération peut permettre de traiter par exemple une déformation inter-phalangienne, ou des orteils en griffe ou en marteau.

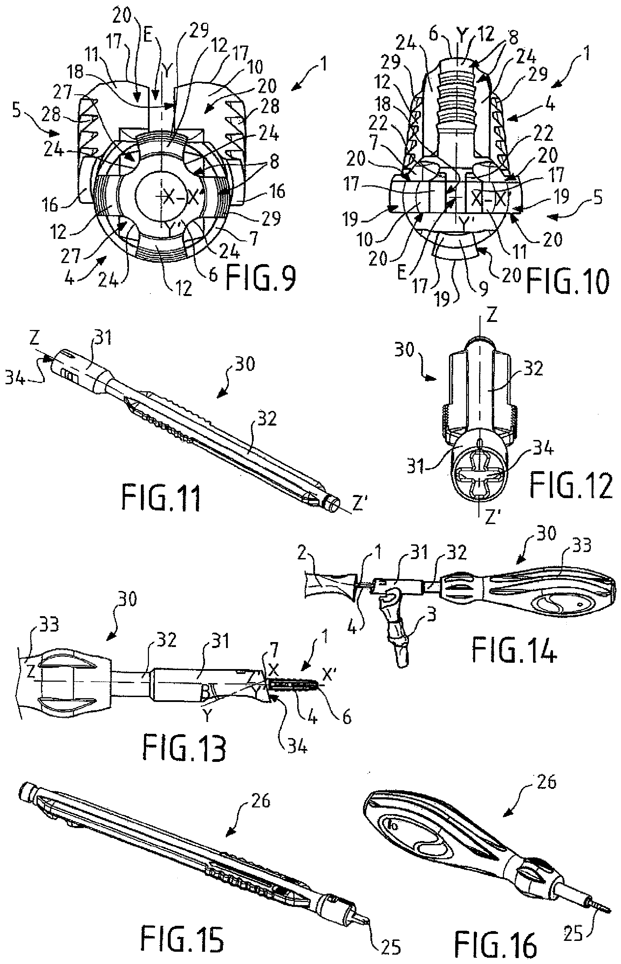

Ainsi, l'implant 1 de l'invention peut avantageusement être inséré au niveau de l'articulation entre deux os, par exemple une articulation entre deux os de la colonne vertébrale, du genou, ou du coude. De préférence, l'implant 1 de l'invention est un implant 1 phalangien, le premier os 2 formant une première phalange et le deuxième os 3 formant une deuxième phalange du même doigt que la première phalange, tel qu'illustré à titre d'exemple aux figures 13 et 14. Les phalanges concernées sont par exemple situées dans un pied du patient, ou dans une main de ce dernier. Une fois l'arthrodèse effectuée, l'articulation entre les deux phalanges concernées du même doigt ou orteil est avantageusement bloquée, de sorte que ledit doigt ou orteil n'est plus capable d'être plié au niveau de cette articulation en particulier.

L'implant 1 est avantageusement prévu pour être laissé dans le corps du patient à l'issue de l'opération, tout au long de la vie dudit patient, et pour ne pas nécessiter une ablation dudit implant 1 une fois l'arthrodèse effectuée. Bien entendu, l'implant 1 pourra être conçu au contraire pour pouvoir être retiré du corps du patient à l'issue de l'arthrodèse.

Tel qu'illustré aux figures 1 à 10, l'implant 1 de l'invention comprend un corps d'ancrage 4 conçu pour assurer l'ancrage de l'implant 1 dans le premier os 2, et une portion déformable 5 conçue pour assurer l'ancrage de l'implant 1 dans le deuxième os 3. Le corps d'ancrage 4 et la portion déformable 5 sont solidaires l'un de l'autre et disposés de préférence en opposition l'un par rapport à l'autre, par exemple de part et d'autre d'un plan intermédiaire ou médian. L'implant 1 de l'invention peut ainsi être ancré d'une part dans le premier os 2 par l'intermédiaire du corps d'ancrage 4, et d'autre part dans le deuxième os 3 par l'intermédiaire de la portion déformable 5, de sorte que le premier os 3 et le deuxième os 4 sont reliés l'un à l'autre par l'implant 1. Le corps d'ancrage 4, et respectivement la portion déformable 5, est avantageusement conçu pour être planté dans l'os concerné, ou inséré en force, à la manière d'une cheville de fixation. Une fois l'implant 1 ancré dans le premier os 2 et dans le deuxième os 3, ces derniers deviennent solidaires l'un par rapport à l'autre par l'intermédiaire de l'implant 1, de façon souple ou rigide, de sorte qu'il se produit une ostéosynthèse conduisant à la fusion osseuse desdits premier et deuxième os 3. De préférence, l'ancrage du corps d'ancrage 4 est effectué par impaction, ou par coincement en force, de ce dernier dans le premier os 2, à la manière d'une cheville. Le corps d'ancrage 4 est avantageusement conçu pour être ancré ainsi, et est par exemple dépourvu de moyens de vissage dudit corps d'ancrage 4 dans le premier os 2. De même, l'ancrage de la portion déformable 5, est préférentiellement effectué par impaction, ou par coincement en force, de cette dernière dans le deuxième os 3. La portion déformable 5 est ainsi avantageusement dépourvue de moyens de vissage de cette dernière dans le deuxième os 3.

De préférence, le corps d'ancrage 4, tout comme la portion déformable 5, sont prévus pour être insérés dans le canal médullaire respectivement du premier os 2 et du deuxième os 3, de sorte que l'implant 1 de l'invention forme préférentiellement un implant 1 intra-médullaire. En l'espèce, le corps d'ancrage 4 est préférentiellement destiné à être ancré dans un canal médullaire du premier os 2, la portion déformable 5 étant destinée à être ancrée dans le canal médullaire du deuxième os 3. Le corps d'ancrage 4 et la portion déformable 5 peuvent dans ce cas être implantés dans les canaux médullaires du premier et du deuxième os 3, par exemple après que l'extrémité de l'os concerné (formée par exemple par une surface cartilagineuse extrémale) ait été percée ou retirée dudit os afin de ménager une ouverture d'accès audit canal médullaire. Le stock osseux intérieur du canal médullaire du premier os 2, respectivement du deuxième os 3, doit également préférentiellement être aménagé pour permettre l'accueil du corps d'ancrage 4. Les figures 13 et 14 représentent à ce propos des exemples d'instruments d'ouverture 26 d'un canal médullaire d'un os préalablement à l'ancrage de l'implant 1 de l'invention dans le premier et le deuxième os 3. Ces instruments d'ouverture 26 sont préférentiellement pourvus d'un embout de perçage 25 de l'os. D'autres instruments ou outils pourront bien entendu être utilisés.

Bien entendu, sans sortir du cadre de l'invention, le corps d'ancrage 4 et/ou la portion déformable 5 pourront être conçus pour être ancrés dans toute autre partie de l'os, et par exemple pour être ancrés dans un orifice ménagé dans la partie corticale de l'os.

Dans le cas préférentiel où l'implant 1 est intra-médullaire, le corps d'ancrage 4, et respectivement la portion déformable 5 sont avantageusement conformés pour s'insérer axialement dans le canal médullaire du premier os 2, respectivement du deuxième os 3. Le corps d'ancrage 4 est ainsi de préférence conçu pour être ancré dans le premier os 2 de façon coaxiale avec ce dernier, et la portion déformable 5 dans le deuxième os 3 de façon coaxiale avec ce dernier. En particulier, le canal médullaire de tels os étant de forme générale tubulaire, sensiblement cylindrique, le corps d'ancrage 4 et la portion déformable 5 auront préférentiellement une forme générale allongée et axiale, et de préférence cylindrique, conique, prismatique ou cruciforme, tel qu'illustré aux figures 1 à 10, et seront conformés pour être insérés de façon coaxiale avec la forme tubulaire du canal médullaire concerné, de manière à pouvoir être coincés au sein du canal médullaire. Notamment dans le cas préférentiel ou les premier os 2 et le deuxième os 3 sont des phalanges, le canal médullaire présente avantageusement un rétrécissement, formant par exemple l'isthme de la phalange, qui permet d'appliquer une pression centripète de coincement de l'implant 1 au sein du canal médullaire, et en particulier du corps d'ancrage 4 et/ou de la portion déformable 5, cette pression centripète permettant de retenir par adhérence l'implant 1 au sein du canal médullaire. L'implant 1, son corps d'ancrage 4 et sa portion déformable 5, seront préférentiellement choisis, conformés, et dimensionnés pour s'adapter à la morphologie des os dans lesquels ils sont ancrés, et éventuellement en fonction de la morphologie du patient.

Tel qu'illustré aux figures 1 à 10, le corps d'ancrage 4 de l'invention s'étend entre une extrémité de pénétration 6 dans ledit premier os 2 et une extrémité de base 7 opposée, et présente ainsi une forme allongée, de préférence le long d'un axe X-X'. L'extrémité de pénétration 6 forme une extrémité libre de l'implant 1, et est avantageusement effilée, conique, bombée ou chanfreinée pour faciliter sa pénétration dans le premier os 2. L'extrémité de base 7 opposée forme un point d'attache du corps d'ancrage 4 avec le reste de l'implant 1, et en particulier avec la portion déformable 5. De préférence, le corps d'ancrage 4 présente une surface externe 8 reliant l'extrémité de base 7 à l'extrémité de pénétration 6, et forme par exemple un corps plein délimité par ladite surface externe 8. Alternativement, tel que décrit ci-après, le corps d'ancrage 4 pourra être canulé. De préférence, la surface externe 8 est de forme généralement convergente, par exemple conique, en direction de l'extrémité de pénétration 6, par exemple le long de l'axe X-X', ce qui permet d'améliorer le coincement du corps d'ancrage 4 dans le premier os 2, le corps d'ancrage 4 pouvant ainsi par exemple s'adapter à plusieurs diamètres de canaux médullaires. Le corps d'ancrage 4 présente ainsi préférentiellement une section de taille plus importante par exemple en longueur, en largeur ou en diamètre à proximité de l'extrémité de base 7 qu'à proximité de l'extrémité de pénétration 6. Alternativement, le corps d'ancrage 4 peut être de forme générale prismatique le long de l'axe X-X', et ne pas être convergent, sans pour autant sortir du cadre de l'invention. Au sens de l'invention, on entend par « *forme générale* » la forme extérieure de l'élément concerné, c'est-à-dire n'incluant pas en particulier les diverses aspérités, rainures, chanfreins, ailettes, gorges, dents, et autres éléments saillants ou rentrants.

Le corps d'ancrage 4 de l'invention est sensiblement rigide et non déformable, ou pour le moins, le corps d'ancrage 4 est moins facilement déformable que la portion déformable 5, et nécessite un effort plus important pour être déformé. Ainsi, le corps d'ancrage 4 est avantageusement conçu pour être ancré dans le premier os 2, notamment dans le cas où ce dernier forme un os plus résistant ou d'une dureté supérieure au deuxième os 3. Selon une telle conception, l'implant 1 est particulièrement adapté à la morphologie de l'ossature des patients, dans le cas où les os à fusionner sont de résistance différente, par exemple dans le cas où le premier os 2 forme une phalange proximale, et le deuxième os 3 une phalange distale, c'est-à-dire extrémale, du doigt ou de l'orteil du patient. La portion déformable 5 étant conçue ancrée dans le deuxième os 3 et étant plus déformable que le corps d'ancrage 4, son ancrage n'est pas de nature à détériorer le deuxième os 3, et permet une bonne reconstruction osseuse à la suite de l'insertion de l'implant 1. La tenue dans le premier os 2 du corps d'ancrage 4 est parallèlement particulièrement fiable et solide, et l'implant 1 particulièrement résistant.

En particulier, la structure et/ou le matériau formant le corps d'ancrage 4 sont avantageusement choisis et conçus pour que le corps d'ancrage 4 ne se déforme pas, ou de façon minime, sous l'action du premier os 2, lorsque le corps d'ancrage 4 est ancré dans ledit premier os 2. En particulier, le corps d'ancrage 4 est conçu pour ne pas fléchir le long de l'axe longitudinal X-X', ou pour ne pas se contracter sous l'effet d'une pression axiale ou radiale, lorsqu'il est introduit, et éventuellement ancré, dans le premier os 2. Néanmoins, de façon locale, le corps d'ancrage 4 pourra subir des déformations propres à permettre au maintien de son ancrage dans le premier os 2 ; en particulier, le corps d'ancrage 4 pourra comprendre des éléments de rétention (décrits ci-après) qui peuvent être déformés par le deuxième os 3, en particulier au niveau de l'isthme du canal médullaire, pour permettre une bonne rétention du corps d'ancrage 4 au sein dudit deuxième os 3.

De préférence, la structure et la forme du corps d'ancrage 4 lui confèrent, ou au moins contribuent à lui conférer, son caractère rigide. Pour cela, le corps d'ancrage 4 forme préférentiellement un corps plein et massif (tel qu'illustré aux figures 5 à 10), ou un corps tubulaire (tel qu'illustré aux figures 1 à 4). Le corps d'ancrage 4 comprend de préférence des nervures longitudinales de renfort 12, lesquelles sont par exemple réparties autour de l'axe X-X', faisant saillie de la surface externe 8 dans le sens de la longueur du corps d'ancrage 4, par exemple à partir de l'extrémité de pénétration 6. Les nervures longitudinales de renfort 12, permettent avantageusement de rigidifier le corps d'ancrage 4, en particulier en flexion et en torsion, sans pour autant que ce dernier ne soit d'une épaisseur trop importante.

Selon l'invention, la portion déformable 5 comprend au moins deux bras d'ancrage 9, 10, 11 qui font saillie du corps d'ancrage 4 à partir de l'extrémité de base 7 de ce dernier. Tel qu'illustré aux figures 1 à 10, les bras d'ancrage 9 de l'invention s'élèvent du corps d'ancrage 4, à partir de l'extrémité de base 7, et sont orientés les uns et les autres dans une direction similaire le long d'un axe Y-Y', qui est préférentiellement opposée à la direction du corps d'ancrage 4. L'axe Y-Y' forme ainsi la direction d'extension générale de la portion déformable 5, cette dernière étant le résultat de la combinaison des bras d'ancrage 9, 10, 11, lesquels forment avantageusement un faisceau de bras d'ancrage 9, 10, 11. Chaque bras d'ancrage 9, 10, 11 s'étend avantageusement entre une extrémité de jonction 16 avec le corps d'ancrage 4 et une extrémité terminale 17 opposée d'implantation dudit bras d'ancrage 9 dans le deuxième os 3, le long d'un axe d'extension propre.

De préférence, les bras d'ancrage 9, 10, 11 présentent une section orthogonale à leur axe d'extension propre qui est moins importante que la section orthogonale à l'axe X-X' du corps d'ancrage 4. Les bras d'ancrage 9, 10, 11 sont ainsi préférentiellement plus fins que le corps d'ancrage 4, ce qui leur confère une déformabilité plus importante que celle de ce dernier. Les bras d'ancrage 9, 10, 11 sont donc de préférence flexibles, de façon élastique ou élastico-plastique, afin que la portion déformable 5 qu'ils forment soit globalement plus facile à déformer que le corps d'ancrage 4. La portion déformable 5 peut ainsi avantageusement être ancrée dans le deuxième os 3, en particulier lorsque ce dernier est plus mou, plus fragile et/ou plus fin que le premier os 2. De plus, la portion déformable 5 s'adapte, par déformation, à la forme du deuxième os 3, et en particulier de son canal médullaire, afin de pouvoir être ancrée de façon fiable, sûre, sans douleur pour le patient. L'implant 1 est ainsi particulièrement adapté aux caractéristiques mécaniques et morphologiques, à la fois du premier os 2 et du deuxième os 3, de sorte que sa liaison avec ces derniers est particulièrement fiable, la douleur subie par le patient relativement faible, et la durée du traitement réduite. La déformabilité et la souplesse des bras d'ancrage 9, 10, 11 confère en outre à la liaison entre le premier os 2 et le deuxième os 3 formée par l'implant 1 une certaine souplesse qui est de nature à stimuler l'ostéosynthèse naturelle, et accélérer la fusion osseuse.

La variante préférentielle de l'invention représentée aux figures 1 à 4 forme un implant 1 droit, c'est-à-dire dans lequel, de préférence, le corps d'ancrage 4 s'étend le long d'un axe X-X', la portion déformable 5 s'étendant dans une direction opposée du corps d'ancrage 4 le long d'un axe Y-Y' coaxial avec l'axe X-X'. Un tel implant 1 est particulièrement adapté dans le cas où le premier os 2 et le deuxième os 3 doivent être disposés de façon coaxiale, de préférence de façon à ce que leurs canaux médullaires soient coaxiaux.

Une autre variante préférentielle de l'invention représentée aux figures 5 à 10 forme un implant 1 coudé, c'est-à-dire dans le quel, de préférence, le corps d'ancrage 4 s'étend le long d'un axe X-X' et la portion déformable 5 s'étendant dans une direction opposée au corps d'ancrage 4 le long d'un axe Y-Y' sécant à l'axe X-X', de sorte que l'axe Y-Y' est incliné par rapport à l'axe X-X' selon un angle d'élévation α compris entre :
- 8 et 12 degrés, de préférence environ 10 degrés (tel qu'illustré aux figures 5 à 7), ou
- 15 et 19 degrés, de préférence environ 17 degrés (tel qu'illustré aux figures 8 à 10).

Un tel implant 1 est particulièrement adapté dans le cas où le premier os 2 et le deuxième os 3 doivent être disposés de façon sécante, par exemple légèrement sécante, selon ce même angle d'élévation a, de préférence de façon à ce que leurs canaux médullaires soient sécants selon ce même angle d'élévation a. Bien entendu l'implant 1 pourra être conçu avec une valeur d'angle d'élévation α différente en fonction de la morphologie osseuse et de l'orientation désirée entre le premier os 2 et le deuxième os 3. De préférence, la valeur de l'angle α correspond à une valeur anatomique de l'orientation naturelle du premier os 2 par rapport au deuxième os 3.

De façon préférentielle, l'implant 1 forme une pièce monobloc d'un seul tenant, la portion déformable 5 venant de matière avec le corps d'ancrage 4. En particulier, les bras d'ancrage 9, 10, 11 prolongent préférentiellement le corps d'ancrage 4 et viennent de matière avec ce dernier de façon à ce que l'implant 1 soit formé d'une seule pièce, ce qui confère une grande résistance structurelle à l'implant 1, et facilite sa fabrication, laquelle peut être effectuée par exemple par moulage et usinage, sans étape d'assemblage de pièces rapportées les unes aux autres. L'implant 1 peut être avantageusement réalisé à l'aide d'une seule matière, la déformabilité des différents éléments qui le constituent étant obtenue à l'aide de la forme de ces derniers, et en particulier à l'aide de leur section et/ou de leur épaisseur. L'implant 1 est préférentiellement réalisé dans un matériau polymère, par exemple du PEEK, ce qui lui permet par exemple d'être à la fois radio-transparent, biocompatible, léger et peu coûteux à fabriquer. Bien entendu, sans sortir du cadre de l'invention, l'implant 1 pourra être réalisé dans un autre matériau, par exemple un matériau métallique de type inox ou titane. Également, l'implant 1 pourra être le résultat de l'assemblage de plusieurs pièces, et ne pas être monobloc.

Selon l'invention, les bras d'ancrage 9, 10, 11 sont séparés l'un de l'autre par un espace libre E de manière à pouvoir être rapprochés l'un de l'autre par déformation desdits bras d'ancrage 9, 10, 11 sous l'action du deuxième os 3. Les bras d'ancrage 9, 10, 11 de l'invention ne se touchent ainsi sensiblement pas, du moins lorsqu'ils ne sont pas déformés, un espace étant avantageusement ménagé entre chacun d'eux, sur au moins une portion de leur longueur, sinon sur toute leur longueur. Les bras d'ancrage 9, 10, 11 disposent ainsi entre eux d'un espace central libre, de manière à pouvoir être déformés de manière à envahir cet espace libre E. Une telle conception rend préférentiellement la portion déformable 5 compressible de façon centripète par rapport à l'axe Y-Y', et/ou apte à être tordue autour dudit axe Y-Y', et/ou apte à être fléchie, les bras d'ancrage 9 étant de préférence libres d'occuper, au moins en partie, l'espace libre E qui les sépare, sous l'action du deuxième os 3 lorsqu'ils sont ancrés dans ce dernier. L'élasticité des bras d'ancrage 9, 10, 11 permet préférentiellement à la portion déformable 5 d'appliquer une pression centrifuge au canal médullaire (ou de façon générale au logement d'ancrage), ce qui est de nature à contribuer à retenir l'implant 1 au sein du deuxième os 3 par exemple par adhérence. De préférence, les bras d'ancrage 9, 10, 11 sont conçus pour évoluer entre une configuration non déformée, qui constitue leur position initiale, et dans laquelle l'espace libre E est effectivement libre, et une position déformée dans laquelle ils sont rapprochés les uns des autres, voire se touchent, et envahissent l'espace libre E initial. De préférence, les bras d'ancrage 9, 10, 11 sont déformables de façon élastique, de manière à revenir d'eux-mêmes sensiblement vers leur configuration non déformée lorsqu'ils en sont écartés.

La portion déformable 5 forme ainsi une liaison fiable, souple et polyvalente, de sorte que l'implant 1 peut être ancré à la fois à un premier os 2 et à un deuxième os 3 qui sont de nature, de résistance et de forme différentes.

De préférence, la portion déformable 5 est formée par trois bras d'ancrage 9, 10, 11, tel qu'illustré aux figures, de manière à améliorer la stabilité de l'implant 1 dans le deuxième os 3 selon trois degrés de liberté. Bien entendu, la portion déformable 5 pourra comporter deux bras d'ancrage, ou quatre bras d'ancrage, par exemple entre deux et huit bras d'ancrage, ou davantage, sans sortir du cadre de l'invention.

De préférence, les bras d'ancrage 9, 10, 11 sont sensiblement parallèles entre eux, par exemple en configuration non déformée, de sorte que leur axe d'extension propre est parallèle à l'axe Y-Y', de manière à pouvoir être déformés par exemple de façon à converger les uns vers les autres, ou pour le moins que leurs extrémités terminales 17 convergent.

Alternativement, les bras d'ancrage 9, 10, 11 peuvent avantageusement être convergents en configuration non déformée, par exemple légèrement convergents, ou au contraire divergents. Dans le cas où les bras d'ancrage 9, 10, 11 sont convergents, la portion déformable 5 forme par exemple un faisceau de bras convergent le long de l'axe Y-Y'.

Dans le cas préférentiel illustré aux figures, les extrémités de jonction 16 des trois bras d'ancrage 9, 10, 11 sont de préférence disposées en Y (tel qu'illustré à la figure 4) ou en T (tel qu'illustré à la figure 10) autour de l'axe Y-Y', sur l'extrémité de base 7 du corps d'ancrage 4, de sorte que les extrémités terminales 17 sont également respectivement disposées en Y ou en T.

De préférence, dans le cas de la variante coudée de l'implant 1 représentée aux figures 5 à 10, les extrémités de jonction 16 des bras d'ancrage 9, 10, 11 sont disposées en T.

Pour le cas de la variante rectiligne de l'implant 1 représentée aux figures 1 à 4, les extrémités de jonction 16 des bras d'ancrage sont disposées en Y.

On entend par « *disposés en T* » que deux des bras d'ancrage 10, 11 sont disposés en opposition de part et d'autre de l'axe Y-Y' de manière à s'étendre le long d'un plan médian commun porté par ledit axe Y-Y', alors que le bras d'ancrage 9 restant est disposé dans un plan médian orthogonal audit plan médian commun et porté par l'axe Y-Y'. Dans ce cas, l'axe Y-Y' forme l'intersection du plan médian commun et du plan orthogonal.

On entend par « *disposés en Y* » que deux des bras d'ancrage 10, 11 sont disposés de part et d'autre de l'axe Y-Y' de manière à s'étendre le long d'un plan médian commun parallèle à l'axe Y-Y' et écarté de ce dernier, alors que le bras d'ancrage 9 restant est disposé dans un plan médian orthogonal audit plan médian commun et porté par l'axe Y-Y'. Dans ce cas, l'axe Y-Y', ne forme pas l'intersection du plan médian commun et du plan orthogonal les bras d'ancrage 9, 10, 11 sont répartis autour dudit axe Y-Y'.

Dans cette configuration, deux des bras d'ancrage 10, 11 sont préférentiellement disposés symétriquement par rapport à un plan porté par l'axe Y-Y', le troisième bras d'ancrage 9 étant porté par ce même plan de symétrie, et étant le troisième bras d'ancrage 9 étant lui-même, dans sa forme, symétrique par rapport audit plan de symétrie. De préférence, l'implant 1 présente sensiblement un plan de symétrie porté par l'axe Y-Y' et l'axe d'extension propre du troisième bras d'ancrage 9. Deux des bras d'ancrage 10, 11 sont préférentiellement conçus et conformés pour pouvoir être déformés de manière privilégiée en direction l'un de l'autre dans un plan commun, de façon à former une pince. Dans ce cas préférentiel, le dernier bras d'ancrage 9 est avantageusement conçu et conformé pour pouvoir être déformé de façon centripète à l'axe Y-Y'. Une telle conception permet d'améliorer la stabilité de l'implant 1 au sein du deuxième os 3, et la fiabilité de son ancrage.

De préférence, au moins l'un des bras d'ancrage 9, 10, 11 présente une section dont la forme générale est sensiblement trapézoïdale. Ainsi, l'un des bras d'ancrage 9, 10, 11, au moins, de préférence tous les bras d'ancrage 9, 10, 11, présente une forme générale prismatique de bas trapézoïdale, ce qui permet de favoriser sa flexibilité selon des directions prédéterminées, par exemple en direction de l'axe Y-Y'. En effet, la distance entre les côtés opposés du trapèze formant la section des bras d'ancrage 9, 10, 11 peut être choisie de manière à être moins élevée dans la direction de déformation, et plus élevée dans la direction transversale à la déformation.

Par exemple, la section du bras d'ancrage 9 représenté aux figures permet de former un bras d'ancrage 9 comprenant :
- un côté intérieur 18 formant une face orientée en direction de l'axe Y-Y' et sensiblement ortho-radiale à ce dernier,
- un côté extérieur 19 opposé, lui aussi sensiblement ortho-radial à l'axe Y-Y', et
- deux côtés latéraux 20 reliant le côté extérieur 19 au côté intérieur 18, et étant par exemple radiaux à l'axe Y-Y', ou pour le moins sécants au côté intérieur 18 et au côté extérieur 19.

De préférence, le côté intérieur 18 est séparé du côté extérieur 19 par une distance radiale D_{R} inférieure à une distance ortho-radiale D_{O} séparant les côtés latéraux 20, de sorte que la flexion radiale à l'axe Y-Y' du bras d'ancrage 9 est facilitée par rapport à une flexion ortho-radiale à l'axe Y-Y'.

Cette forme particulière fait que les bras d'ancrage 9, 10, 11 présentent également des arêtes longitudinales propres à bloquer l'implant 1 en rotation autour de l'axe Y-Y' par rapport au deuxième os 3.

Le terme « *forme générale sensiblement trapézoïdale* » comprend avantageusement le cas où certains côtés du trapèze sont courbes, notamment le côté intérieur 18 et/ou le côté extérieur 19. En particulier, le côté extérieur 19 des bras d'ancrage 9, 10, 11 peut avantageusement être courbe. De préférence, le côté extérieur 19 de la forme générale trapézoïdale du bras d'ancrage 9, 10, 11 forme avantageusement un arc de cercle dont le centre est par exemple formé par l'axe Y-Y', de manière à ce que le contour extérieur de la section orthogonale à l'axe Y-Y' de la portion déformable 5 forme sensiblement un cercle centré sur ledit axe Y-Y' (tel qu'illustré aux figures 4 et 10). En configuration non déformée, les bras d'ancrage 9, 10, 11 sont préférentiellement sensiblement parallèles entre eux et à l'axe Y-Y', de sorte que la portion déformable 5 présente un contour extérieur, qui est par exemple formé l'union des côtés extérieurs 19 des bras d'ancrage 9, 10, 11, de forme générale sensiblement cylindrique ou prismatique autour de l'axe Y-Y'.

De façon préférentielle, en configuration déformée, les bras d'ancrage 9, 10, 11, qui étaient initialement parallèles, convergent, et voient par exemple leurs extrémités terminales 17 resserrés autour de l'axe Y-Y', de sorte que le contour extérieur de la portion déformable 5 converge, et est par exemple de forme générale sensiblement tronconique.

Le côté intérieur 18 peut quant à lui être préférentiellement courbe, de façon à ce que la face intérieure des bras d'ancrage 10, 11 forme une portion de cylindre ou de cône concave d'axe Y-Y', de sorte qu'une portion de l'espace libre E formé par les bras d'ancrage 10, 11 est sensiblement cylindrique ou tronconique, tel que cela est visible notamment à la figure 4 et à la figure 10.

L'implant 1 comprend préférentiellement une canule 23 traversant le corps d'ancrage 4 de manière à prolonger ledit espace libre E jusqu'à l'extrémité de pénétration 6. Ainsi, de préférence, la canule 23 est prolongée par l'espace libre E délimité par les côtés intérieurs 18 des bras d'ancrage 9, 10, 11, de sorte que l'implant 1 peut être enfilé à coulissement, par l'intermédiaire de sa canule 23 et de son espace libre E, sur une tige de guidage, ou une broche de guidage, ce qui facilite la mise en place de l'implant 1 dans les os du patient. Les côtés intérieurs 18 des bras d'ancrage 9, 10, 11 forment avantageusement une prolongation de la canule 23 qui est de forme sensiblement cylindrique de diamètre supérieur au diamètre de ladite canule 23, de manière à ce que les bras d'ancrage 9, 10, 11 disposent d'un jeu formé par l'espace libre E résiduel ménagé entre la broche de guidage et le côté intérieur 18, pour pouvoir se déformer en direction de l'axe Y-Y' malgré la présence de ladite broche de guidage. De façon préférentielle, l'implant 1 ne comprend une canule 23 que s'il forme un implant 1 droit tel qu'illustré par exemple aux figures 1 à 4, et non s'il forme un implant coudé tel qu'illustré par exemple aux figures 5 à 10. Ainsi, la tige de guidage, optionnellement rigide et rectiligne, peut être insérée dans le long de la canule 23, de l'espace libre E, et des axes X-X' et Y-Y' qui sont coaxiaux et dans l'alignement les uns des autres.

De préférence, tel qu'illustré à la figure 4, les côtés latéraux 20 de deux des bras d'ancrage 10, 11 sont parallèles entre eux, et deux à deux coplanaires, de sorte que la forme des deux bras d'ancrage 10, 11 concernés est délimitée par deux plans parallèles communs auxdits deux bras d'ancrage 10, 11.

Par ailleurs, tel qu'illustré par exemple aux figures 5 à 10, au moins l'un des bras d'ancrage 9, 10, 11 est pourvu d'une ailette longitudinale 22 de renfort faisant saillie du bras d'ancrage 9, 10, 11 à partir d'un côté latéral 20 de ce dernier, tel que cela est particulièrement visible à la figure 10. De préférence, l'implant 1 comprend deux ailettes longitudinales 22, portées respectivement par deux bras d'ancrage 10, 11 dont les côté latéraux 20 sont par exemple sensiblement coplanaires, les ailettes longitudinales 22 s'élevant parallèlement l'une à l'autre dans la direction de l'axe Y-Y'. Les ailettes longitudinales 22 permettent avantageusement à la fois de rigidifier le bras d'ancrage concerné, mais aussi par exemple de contribuer à permettre un blocage en rotation au tour de l'axe Y-Y' de l'implant 1 par rapport au deuxième os 3 lorsqu'il est ancré dans ce dernier.

Au moins l'un des bras d'ancrage 9, 10, 11, de préférence deux des bras d'ancrage 10, 11, voire tous, présente une zone de section réduite 21 de manière à favoriser la déformation dudit bras d'ancrage 9, 10, 11 par flexion dudit bras d'ancrage au niveau de la zone de section réduite 21, en direction ou à l'écart d'un autre des bras d'ancrage 9, 10, 11. Tel qu'illustré aux figures 5 à 10, la zone de section réduite 21 forme de préférence une encoche ménagée dans le bras d'ancrage 9, 10, 11, au voisinage de l'extrémité de jonction 16, l'encoche étant avantageusement ouverte sur l'espace libre E et tournée en direction d'un autre des bras d'ancrage 9, 10, 11 ou de l'axe Y-Y', par exemple de façon à ce que le bras d'ancrage 9, 10, 11 concerné puisse être fléchi au niveau de sa section réduite 21 de manière à converger vers un autre des bras d'ancrage 9, 10, 11. Chaque bras d'ancrage 9, 10, 11, de préférence deux d'entre eux, peut ainsi être conçu pour être sensiblement rigide sur la majorité de sa longueur, et ne se déformer par exemple qu'au niveau de sa section réduite 21 lorsqu'il est sollicité par le deuxième os 3.

Le corps d'ancrage 4 s'étendant le long d'un axe X-X' depuis l'extrémité de base 7 jusqu'à l'extrémité de pénétration 6, ledit corps d'ancrage 4 comprend au moins un sillon longitudinal 24 périphérique permettant de bloquer l'implant 1 en rotation autour de l'axe X-X' au sein du premier os 2, le sillon longitudinal 24 s'étendant de façon sensiblement parallèle à l'axe X-X' à partir de l'extrémité de pénétration 6 sur au moins une portion de la longueur du corps d'ancrage 4. L'implant 1 comprend, de manière avantageuse, quatre sillons longitudinaux 24 périphériques, de sorte que le corps d'ancrage 4 présente une section orthogonale sensiblement cruciforme à partir de l'extrémité de pénétration 6 sur au moins une portion de sa longueur. De préférence, la géométrie d'au moins deux sillons 24 périphériques est symétriques par rapport à l'axe X-X', tel que cela est bien visible à la figure 3.

Les sillons longitudinaux 24 sont avantageusement disposés dans le prolongement des espaces ménagés entre les bras d'ancrage 9, 10, 11, et en particulier de manière à prolonger les plans d'extensions associés aux côtés latéraux 20 des bras d'ancrage 9, 10, 11. Alternativement, les sillons longitudinaux 24 peuvent au contraire être disposés dans le prolongement des bras d'ancrage 9, 10, 11. Les sillons longitudinaux 24 pourront également être disposés de façon à ne pas être alignés ni avec les bras d'ancrage 9, 10, 11, ni avec les espaces ménagés entre lesdits bras d'ancrage 9, 10, 11.

Les sillons longitudinaux 24 sont préférentiellement séparés par les nervures longitudinales de renfort 12, qui sont par exemple alignées chacune avec l'un des bras d'ancrage 9, 10, 11, afin de s'étendre en particulier le long d'un axe d'extension commun entre le bras d'ancrage et la nervure longitudinale de renfort associée. La section orthogonale du corps d'ancrage 4 est ainsi avantageusement en forme de croix, dont chacune des branches est formée par l'une des nervures longitudinales 12.

L'implant 1 pourra comprendre plus, ou moins de sillons longitudinaux 24 séparés par les nervures longitudinales 12, par exemple en fonction du nombre de bras d'ancrage 9, 10, 11 envisagé, de manière à ce que le corps d'ancrage 4 présente une section orthogonale en étoile dont chacune des branches est formée par l'une desdites nervures longitudinales 12.

De préférence, les sillons longitudinaux 24 et les nervures longitudinales 12 associées s'étendent sur une portion seulement du corps d'ancrage 4 à partir de l'extrémité de pénétration 6, de sorte que lesdits sillons longitudinaux 24 s'étendent entre ladite extrémité de pénétration 6 et une extrémité de butée 27 desdits sillons longitudinaux 24 située entre ladite extrémité de pénétration 6 et l'extrémité de base 7. Selon cette configuration préférentielle, l'extrémité de butée 27 des sillons longitudinaux 24 permet d'arrêter l'implant 1 en translation le long de l'axe X-X' lors de son ancrage dans le premier os 2, lorsque ce dernier entre en contact avec ladite extrémité de butée 27.

Le bord des sillons longitudinaux 24 forme de préférence une arête saillante, tel qu'illustré aux figures 1 à 10, de manière à améliorer le blocage en rotation autour de l'axe X-X' de l'implant 1 dans le premier os 2.

Par ailleurs, l'implant 1 est préférentiellement muni d'éléments de rétention dudit implant 1 dans les os.

Au moins l'un des bras d'ancrage 9, 10, 11, de préférence chacun des bras d'ancrage 9, 10, 11, est avantageusement pourvu d'un élément de rétention primaire 28 dudit bras d'ancrage 9, 10, 11 dans le deuxième os 3.

L'élément de rétention primaire 28 est préférentiellement formé par une ligne de dents primaires de rétention s'étendant le long du bras d'ancrage 9, 10, 11, et faisant saillie de ce dernier de façon centrifuge par rapport l'espace libre E.

Tel qu'illustré aux figures 1 à 10, les dents primaires de rétention sont disposées au sur le côté extérieur 19 et s'élèvent radialement par rapport à l'axe Y-Y'. L'élément de rétention primaire 28 est conçu pour s'opposer au retrait de l'implant 1 du deuxième os 3 par exemple par accrochement desdites dents primaires contre la paroi intérieure du canal médullaire, tout en ne s'opposant sensiblement pas à l'insertion de l'implant 1 dans ledit deuxième os 3. Pour cela, les dents primaires présentent de préférence une face à pente douce, de faible inclinaison par rapport à l'axe Y-Y', dirigée vers l'extrémité de terminale 17, et une face à pente forte, de forte inclinaison par rapport à l'axe Y-Y', par exemple orthogonale à l'axe X-X' en direction de l'extrémité de jonction 16.

Le corps d'ancrage 4 comprend avantageusement au moins un élément de rétention secondaire 29 du corps d'ancrage 4 dans le premier os 2, qui est préférentiellement formé par une ligne de dents secondaires de rétention s'étendant le long du corps d'ancrage 4. Tel qu'illustré aux figures 1 à 10, les dents secondaires de rétention sont disposées au sommet des nervures longitudinales 12 et s'élèvent radialement par rapport à l'axe X-X'. L'élément de rétention secondaire 29 est conçu pour s'opposer au retrait de l'implant 1 du premier os 2 par exemple par accrochement desdites dents secondaires contre la paroi intérieure du canal médullaire, tout en ne s'opposant sensiblement pas à l'insertion de l'implant 1 dans ledit premier os 2. Pour cela, les dents secondaires présentent de préférence une face à pente douce, de faible inclinaison par rapport à l'axe X-X', dirigée vers l'extrémité de pénétration 6, et une face à pente forte, de forte inclinaison par rapport à l'axe X-X', par exemple orthogonale à l'axe X-X' en direction de l'extrémité de base 7.

Ainsi, l'ancrage de l'implant 1 dans les os du patient est ainsi particulièrement fiable et durable, tout en étant facile à effectuer.

L'invention concerne également, bien que non revendiqué en tant que tel, un instrument de préhension 30 d'un implant 1 tel que décrit précédemment, l'instrument de préhension 30 comprenant un embout de solidarisation 31 amovible de l'implant 1 avec l'instrument de préhension 30, par l'intermédiaire de la portion déformable 5 dudit implant 1. Un exemple de réalisation d'un tel instrument de préhension 30 est illustré aux figures 11 à 14.

Cet instrument de préhension 30 est avantageusement conçu pour permettre la préhension d'un implant 1 droit, tel que décrit précédemment.

L'instrument de préhension 30 comprend préférentiellement un corps principal 32, de forme générale par exemple allongée, s'étendant le long d'un axe Z-Z', et se terminant par ledit embout de solidarisation 31. Ce dernier permet avantageusement de saisir de façon amovible l'implant 1 avec l'instrument de préhension 30 par l'intermédiaire de la portion déformable 5 dudit implant 1. L'embout de solidarisation 31 est préférentiellement conçu pour accueillir la portion déformable 5 dudit implant 1 de façon à ce que l'axe X-X' soit coaxial avec l'axe Z-Z' lorsque l'implant 1 est solidaire de l'instrument de préhension 30. Lorsque l'implant 1 est solidaire de l'embout de solidarisation 31, le corps d'ancrage 4 fait préférentiellement saillie de ce dernier de manière à ce que l'axe X-X' et l'axe Z-Z' soient coaxiaux.

Le chirurgien peut ainsi avantageusement saisir l'implant 1 par l'intermédiaire de l'instrument de préhension 30 afin de manoeuvrer ledit implant 1 pour l'ancrer au premier os 2 et/ou au deuxième os 3.

L'embout de solidarisation 31 est de préférence de forme générale extérieure cylindrique d'axe Z-Z'. Tel qu'illustré à la figure 12, il comprend de préférence un orifice d'accueil 34 de la portion déformable 5 de l'implant 1, ledit orifice d'accueil étant préférentiellement forme cruciforme, ou par exemple en étoile, afin de bloquer l'implant 1 en rotation autour de l'axe Z-Z' par rapport à l'outil de préhension 30. La forme cruciforme ou en étoile correspond avantageusement au contour extérieur de la portion déformable 5 de l'implant 1, et en particulier à la disposition des bras d'ancrage 9, 10, 11, lesquels peuvent s'insérer dans les quatre encoches ou branches de la forme cruciforme afin de pouvoir être entraînés en rotation autour de l'axe Z-Z' par le chirurgien, par l'intermédiaire de l'instrument de préhension 30.

L'instrument de préhension 30 comprend de préférence un manche amovible 33 connectable de façon amovible et solidaire au corps principal 32. Le manche amovible 33 peut avantageusement constituer une zone de préhension privilégiée de l'instrument de préhension 30 par le chirurgien. Alternativement, le corps principal 32 peut être connecté à une machine afin de manoeuvrer l'implant par l'intermédiaire de ladite machine.

L'invention concerne enfin, bien que non revendiqué en tant que tel, un instrument de préhension 30 d'un implant 1 tel que décrit ci-avant, et en particulier dont le corps d'ancrage 4 s'étend le long d'un axe X-X' la portion déformable 5 s'étendant dans une direction opposée au corps d'ancrage 4 le long d'un axe Y-Y' sécant à l'axe X-X', de sorte que l'axe Y-Y' est incliné par rapport à l'axe X-X' selon un angle d'élévation α compris entre :
- 8 et 12 degrés, de préférence environ 10 degrés, ou
- 15 et 19 degrés, de préférence environ 17 degrés.

Ainsi, cet instrument de préhension 30 peut avantageusement être utilisé pour effectuer la préhension d'un implant 1 coudé, tel que décrit précédemment.

L'instrument de préhension 30 comprend un corps principal 32 s'étendant le long d'un axe Z-Z' et se terminant par un embout de solidarisation 31 amovible de l'implant 1 avec l'instrument de préhension 30, par l'intermédiaire de la portion déformable 5 dudit implant 1, l'embout de solidarisation 31 étant conçu pour accueillir la portion déformable 5 dudit implant 1 de façon à ce que l'axe Y-Y' soit sécant à l'axe Z-Z' selon un angle de compensation β égal à l'angle d'élévation a, de manière à compenser l'inclinaison de l'axe Y-Y' par rapport à l'axe X-X', de sorte que l'axe X-X' est coaxial avec l'axe Z-Z' lorsque l'implant 1 est solidaire de l'instrument de préhension 30.

L'embout de solidarisation 31 est de préférence de forme générale extérieure cylindrique d'axe Z-Z'. Tel qu'illustré à la figure 12, il comprend de préférence un orifice d'accueil 34 de la portion déformable 5 de l'implant 1, ledit orifice d'accueil étant préférentiellement forme cruciforme, ou par exemple en étoile, afin de bloquer l'implant 1 en rotation autour de l'axe Y-Y' et/ou X-X' et/ou Z-Z' par rapport à l'outil de préhension 30. La forme cruciforme ou en étoile correspond avantageusement au contour extérieur de la portion déformable 5 de l'implant 1, et en particulier à la disposition des bras d'ancrage 9, 10, 11, lesquels peuvent s'insérer dans les quatre encoches ou branches de la forme cruciforme afin de pouvoir être entraînés en rotation autour de l'axe Y-Y' et/ou X-X' et/ou Z-Z' par le chirurgien, par l'intermédiaire de l'instrument de préhension 30.

Les caractéristiques de l'instrument de préhension 30 pour un implant 1 droit, dont l'axe X-X' et l'axe Y-Y' sont coaxiaux, s'appliquent de préférence également *mutatis mutandis* à cet instrument de préhension 30 pour implant 1 coudé.

Enfin, un exemple de procédé chirurgical d'implantation de l'implant 1 décrit précédemment dans le corps d'un patient ou d'un animal va être décrit ci-après.

Le procédé comporte notamment les étapes suivantes, préférentiellement effectuées dans l'ordre :
- On effectue une incision dans le corps d'un patient afin d'atteindre le premier os 2 et le deuxième os 3.
- On sectionne l'articulation reliant le premier os 2 au deuxième os 3 afin de dégager les parties extrémales desdits premier os 2 et deuxième os 3.
- On retire l'extrémité desdits premier os 2 et deuxième os 3, par exemple en la perçant, afin de mettre à jour les canaux médullaires desdits premier os 2 et deuxième os 3, par exemple à l'aide d'un instrument d'ouverture 26 représenté aux figures 15 et 16.
- On évide l'intérieur des canaux médullaires de façon partielle ou totale afin de préparer un espace libre pour permettre l'introduction de la portion déformable 5 et du corps d'ancrage 4 de l'implant 1 respectivement dans chacun desdits canaux médullaires.
- On saisit l'implant 1 à l'aide de l'instrument de préhension 30, tel qu'illustré par exemple à la figure 13, de sorte que le corps d'ancrage 4 fasse saillie de l'embout de solidarisation 31, et que les axes X-X' et Z-Z' soient coaxiaux.
- On ancre l'implant 1, optionnellement en force, dans le canal médullaire du premier os 2 (tel qu'illustré à la figure 14), par l'intermédiaire du corps d'ancrage 4.
- On désolidarise l'instrument de préhension 30 de l'implant 1.
- On enfile le deuxième os 3 sur la portion déformable 5 de l'implant 1.

Le procédé pourra également comporter une étape d'ajout d'un matériau ostéo-inducteur dans les canaux médullaires, ou autour de l'implant 1 lorsqu'il est ancré. Certaines étapes du procédé décrites ci-avant pourront avantageusement être omises, ou effectuées dans un ordre différent.

### POSSIBILITE D'APPLICATION INDUSTRIELLE

L'invention trouve son application industrielle dans la conception, la réalisation et la mise en oeuvre d'implants d'arthrodèse permettant de favoriser la fusion osseuse d'un premier os avec un deuxième os.

## Revendications

1. Implant (1) d'arthrodèse permettant de favoriser la fusion osseuse d'un premier os (2) avec un deuxième os (3), ledit implant (1) comprenant :
- un corps d'ancrage (4) sensiblement rigide et non déformable, conçu pour assurer l'ancrage de l'implant (1) dans le premier os (2), le corps d'ancrage (4) s'étendant entre une extrémité de pénétration (6) dans ledit premier os (2) et une extrémité de base (7) opposée, et
- une portion déformable (5) conçue pour assurer l'ancrage de l'implant (1) dans le deuxième os (3), ladite portion déformable (5) comprenant au moins deux bras d'ancrage (9, 10, 11) qui font saillie du corps d'ancrage (4) à partir de l'extrémité de base (7) de ce dernier, les bras d'ancrage (9, 10, 11) étant séparés l'un de l'autre par un espace libre (E) de manière à pouvoir être rapprochés l'un de l'autre par déformation desdits bras d'ancrage sous l'action du deuxième os (3),
et étant **caractérisé en ce qu'**au moins l'un des bras d'ancrage (9, 10, 11) est pourvu d'une ailette longitudinale (22) de renfort faisant saillie du bras d'ancrage (9, 10, 11) à partir d'un côté latéral (20) de ce dernier.

2. Implant (1) selon la revendication précédente, **caractérisé en ce que** la portion déformable (5) est formée par trois bras d'ancrage (9, 10, 11).

3. Implant (1) selon l'une quelconque des revendications précédentes, **caractérisé en ce qu'**au moins l'un des bras d'ancrage (9, 10, 11) présente une section dont la forme générale est sensiblement trapézoïdale, un côté extérieur (19) de la forme générale trapézoïdale formant un arc de cercle de manière à ce que le contour extérieur de la section orthogonale de la portion déformable (5) forme sensiblement un cercle.

4. Implant (1) selon l'une quelconque des revendications précédentes, **caractérisé en ce qu'**au moins l'un des bras d'ancrage (9, 10, 11), de préférence chacun des bras d'ancrage (9, 10, 11), est pourvu d'un élément de rétention primaire (28) dudit bras d'ancrage (9, 10, 11) dans le deuxième os (3).

5. Implant (1) selon la revendication précédente, **caractérisé en ce que** l'élément de rétention primaire (28) est formé par une ligne de dents primaires de rétention s'étendant le long du bras d'ancrage (9, 10, 11), et faisant saillie de ce dernier de façon centrifuge par rapport l'espace libre (E).

6. Implant (1) selon l'une quelconque des revendications précédentes, **caractérisé en ce que** le corps d'ancrage (4) s'étend le long d'un axe (X-X'), la portion déformable (5) s'étendant dans une direction opposée du corps d'ancrage (4) le long d'un axe (Y-Y') coaxial avec l'axe (X-X').

7. Implant (1) selon la revendication précédente, **caractérisé en ce que** chaque bras d'ancrage (9, 10, 11) s'étend entre une extrémité de jonction (16) avec le corps d'ancrage (4) et une extrémité terminale (17) opposée d'implantation dudit bras d'ancrage (9) dans le deuxième os (3), deux des bras d'ancrage (10, 11) étant disposés de part et d'autre de l'axe (Y-Y') de manière à s'étendre le long d'un plan médian commun parallèle à l'axe (Y-Y') et écarté de ce dernier.

8. Implant (1) selon l'une quelconque des revendications 1 à 5, **caractérisé en ce que** le corps d'ancrage (4) s'étend le long d'un axe (X-X') et la portion déformable (5) s'étendant dans une direction opposée au corps d'ancrage (4) le long d'un axe (Y-Y') sécant à l'axe (X-X'), de sorte que l'axe (Y-Y') est incliné par rapport à l'axe (X-X') selon un angle d'élévation (a) compris entre :
- 8 et 12 degrés, de préférence environ 10 degrés, ou
- 15 et 19 degrés, de préférence environ 17 degrés.

9. Implant (1) selon la revendication précédente, **caractérisé en ce que** le corps d'ancrage (4) présente une surface externe (8) reliant l'extrémité de base (7) à l'extrémité de pénétration (6), la surface externe (8) étant de forme généralement convergente, par exemple conique, en direction de l'extrémité de pénétration (6).

10. Implant (1) selon l'une quelconque des revendications précédentes, **caractérisé en ce qu'**il comprend une canule (23) traversant le corps d'ancrage (4) de manière à prolonger ledit espace libre (E) jusqu'à l'extrémité de pénétration (6).

11. Implant (1) selon l'une quelconque des revendications précédentes, **caractérisé en ce que** le corps d'ancrage (4) s'étendant le long d'un axe (X-X') depuis l'extrémité de base (7) jusqu'à l'extrémité de pénétration (6), ledit corps d'ancrage (4) comprend au moins un sillon longitudinal (24) périphérique permettant de bloquer l'implant (1) en rotation autour de l'axe (X-X') au sein du premier os (2), le sillon longitudinal (24) s'étendant de façon sensiblement parallèle à l'axe (X-X') à partir de l'extrémité de pénétration (6) sur au moins une portion de la longueur du corps d'ancrage (4).

12. Implant (1) selon la revendication précédente, **caractérisé en ce qu'**il comprend quatre sillons longitudinaux (24) périphériques, de sorte que le corps d'ancrage (4) présente une section orthogonale sensiblement cruciforme à partir de l'extrémité de pénétration (6) sur au moins une portion de sa longueur.

13. Implant (1) selon l'une quelconque des revendications précédentes, **caractérisé en ce qu'**il forme un implant (1) intra-médullaire phalangien, le premier os (2) formant une première phalange et le deuxième os (3) formant une deuxième phalange du même doigt que la première phalange, le corps d'ancrage (4) étant destiné à être ancré dans un canal médullaire du premier os (2), la portion déformable (5) étant destinée à être ancrée dans le canal médullaire du deuxième os (3).

## Patentansprüche

1. Arthrodeseimplantat (1) zum Fördern der Knochenfusion eines ersten Knochens (2) mit einem zweiten Knochen (3), wobei das Implantat (1) Folgendes umfasst:
- einen im Wesentlichen starren und nicht verformbaren Verankerungskörper (4), der zum Verankern des Implantats (1) in dem ersten Knochen (2) ausgebildet ist, wobei sich der Verankerungskörper (4) zwischen einem Eindringungsende (6) in den ersten Knochen (2) und einem gegenüberliegenden Basisende (7) erstreckt und
- einen verformbaren Teil (5), der zum Verankern des Implantats (1) in dem zweiten Knochen (3) ausgebildet ist, wobei der verformbare Teil (5) mindestens zwei Verankerungsarme (9, 10, 11) aufweist, die aus dem Basisende (7) des Verankerungskörpers (4) herausragen, wobei die Verankerungsarme (9, 10, 11) durch einen Freiraum (E) voneinander derart beabstandet sind, dass sie durch Verformung der Verankerungsarme unter Einwirkung des zweiten Knochens (3) näher zueinander gebracht werden können,
und **dadurch gekennzeichnet, dass** mindestens einer der Verankerungsarme (9, 10, 11) mit einer von dem Verankerungsarm (9, 10, 11) ab einer lateralen Seite (20) desselben vorstehenden Längsverstärkungsrippe (22) versehen ist.

2. Implantat (1) nach dem vorhergehenden Anspruch, **dadurch gekennzeichnet, dass** der verformbare Teil (5) von drei Verankerungsarmen (9, 10, 11) gebildet ist.

3. Implantat (1) nach einem der vorhergehenden Ansprüche, **dadurch gekennzeichnet, dass** mindestens einer der Verankerungsarme (9, 10, 11) einen Abschnitt aufweist, dessen Gesamtform im Wesentlichen trapezförmig ist, wobei eine Außenseite (19) der trapezförmigen Gesamtform derart einen Kreisbogen bildet, dass die Außenkontur des orthogonalen Abschnitts des verformbaren Teils (5) im Wesentlichen einen Kreis bildet.

4. Implantat (1) nach einem der vorhergehenden Ansprüche, **dadurch gekennzeichnet, dass** mindestens einer der Verankerungsarme (9, 10, 11), vorzugsweise jeder der Verankerungsarme (9, 10, 11), mit einem primären Halteelement (28) des Verankerungsarms (9, 10, 11) in dem zweiten Knochen (3) versehen ist.

5. Implantat (1) nach dem vorhergehenden Anspruch, **dadurch gekennzeichnet, dass** das primäre Halteelement (28) durch eine Reihe von primären Haltezähnen gebildet ist, die sich entlang des Verankerungsarms (9, 10, 11) erstrecken und von diesem in Bezug auf den Freiraum (E) zentrifugal vorstehen.

6. Implantat (1) nach einem der vorhergehenden Ansprüche, **dadurch gekennzeichnet, dass** sich der Verankerungskörper (4) entlang einer Achse (X-X') erstreckt, wobei sich der verformbare Teil (5) in einer dem Verankerungskörper (4) entgegengesetzten Richtung entlang einer zu der Achse (X-X') koaxialen Achse (Y-Y') erstreckt.

7. Implantat (1) nach dem vorhergehenden Anspruch, **dadurch gekennzeichnet, dass** sich jeder Verankerungsarm (9, 10, 11) zwischen einem Verbindungsende (16) mit dem Verankerungskörper (4) und einem gegenüberliegenden Ende (17) zum Implantieren des Verankerungsarms (9) in den zweiten Knochen (3) erstreckt, wobei zwei der Verankerungsarme (10, 11) auf beiden Seiten der Achse (Y-Y') derart angeordnet sind, dass sie sich entlang einer gemeinsamen Mittelebene erstrecken, welche parallel zu und von der Achse (Y-Y') beabstandet sind.

8. Implantat (1) nach einem der Ansprüche 1 bis 5, **dadurch gekennzeichnet, dass** sich der Verankerungskörper (4) entlang einer Achse (X-X') erstreckt und der verformbare Teil (5) sich in einer dem Verankerungskörper (4) entgegengesetzten Richtung entlang einer Achse (Y-Y'), die die Achse (X-X') schneidet, derart erstreckt, dass die Achse (Y-Y') in Bezug auf die Achse (X-X') um einen Höhenwinkel (a) zwischen:
- 8 und 12 Grad, vorzugsweise etwa 10 Grad, oder
- 15 und 19 Grad, vorzugsweise etwa 17 Grad geneigt ist.

9. Implantat (1) nach dem vorhergehenden Anspruch, **dadurch gekennzeichnet, dass** der Verankerungskörper (4) eine Außenfläche (8) aufweist, die das Basisende (7) mit dem Eindringungsende (6) verbindet, wobei die Außenfläche (8) eine allgemein konvergierende Form, beispielsweise eine konische Form, in Richtung des Eindringungsendes (6) aufweist.

10. Implantat (1) nach einem der vorhergehenden Ansprüche, **dadurch gekennzeichnet, dass** es eine Kanüle (23) umfasst, die durch den Verankerungskörper (4) verläuft, um den Freiraum (E) bis zu dem Eindringungsende (6) zu erweitern.

11. Implantat (1) nach einem der vorhergehenden Ansprüche, **dadurch gekennzeichnet, dass** sich der Verankerungskörper (4) entlang einer Achse (X-X') von dem Basisende (7) bis zu dem Eindringungsende (6) erstreckt, wobei der Verankerungskörper (4) mindestens eine periphere Längsnut (24) zum Verriegeln des Implantats (1) in Drehung um die Achse (X- X') innerhalb des ersten Knochens (2) aufweist, wobei sich die Längsnut (24) im Wesentlichen parallel zu der Achse (X-X') ab dem Eindringungsende (6) über mindestens einen Teil der Länge des Verankerungskörpers (4) erstreckt.

12. Implantat (1) nach dem vorhergehenden Anspruch, **dadurch gekennzeichnet, dass** es vier periphere Längsnuten (24) aufweist, so dass der Verankerungskörper (4) ab dem Eindringungsende (6) über mindestens einen Teil seiner Länge einen im Wesentlichen kreuzförmigen orthogonalen Querschnitt aufweist.

13. Implantat (1) nach einem der vorhergehenden Ansprüche, **dadurch gekennzeichnet, dass** es ein intramedulläres Phalangealimplantat (1) bildet, wobei der erste Knochen (2) eine erste Phalanx und der zweite Knochen (3) eine zweite Phalanx desselben Fingers wie die erste Phalanx bildet, wobei der Verankerungskörper (4) zur Verankerung in einem Markkanal des ersten Knochens (2) und der verformbare Teil (5) zur Verankerung in dem Markkanal des zweiten Knochens (3) bestimmt ist.

## Claims

1. An arthrodesis implant (1) allowing promoting the bone fusion of a first bone (2) with a second bone (3), said implant (1) comprising:
- a substantially rigid and non-deformable anchoring body (4), designed to ensure the anchoring of the implant (1) in the first bone (2), the anchoring body (4) extending between an end (6) for penetration in said first bone (2) and an opposite base end (7), and
- a deformable portion (5) designed to ensure the anchoring of the implant (1) in the second bone (3), said deformable portion (5) comprising at least two anchoring arms (9, 10, 11) which protrude beyond the anchoring body (4) from the base end (7) of the latter, the anchoring arms (9, 10, 11) being separated from each other by a free space (E) so as to be able to be brought closer to each other by deformation of said anchoring arms under the action of the second bone (3),
and being **characterized in that** at least one of the anchoring arms (9, 10, 11) is provided with a longitudinal reinforcing fin (22) protruding beyond the anchoring arm (9, 10, 11) from a lateral side (20) of the latter.

2. The implant (1) according to the preceding claim, **characterized in that** the deformable portion (5) is formed by three anchoring arms (9, 10, 11).

3. The implant (1) according to any one of the preceding claims, **characterized in that** at least one of the anchoring arms (9, 10, 11) has a section whose general shape is substantially trapezoidal, an external side (19) of the general trapezoidal shape forming an arc of a circle so that the external contour of the orthogonal section of the deformable portion (5) substantially forms a circle.

4. The implant (1) according to any one of the preceding claims, **characterized in that** at least one of the anchoring arms (9, 10, 11), preferably each of the anchoring arms (9, 10, 11), is provided with a primary member (28) for retaining said anchoring arms (9, 10, 11) in the second bone (3).

5. The implant (1) according to the preceding claim, **characterized in that** the primary retention member (28) is formed by a line of primary retention teeth extending along the anchoring arms (9, 10, 11), and protruding centrifugally beyond the latter relative to the free space (E).

6. The implant (1) according to any one of the preceding claims, **characterized in that** the anchoring body (4) extends along an axis (X-X'), the deformable portion (5) extending in an opposite direction of the anchoring body (4) along an axis (Y-Y ') coaxial with the axis (X-X').

7. The implant (1) according to the preceding claim, **characterized in that** each anchoring arms (9, 10, 11) extends between a junction end (16) with the anchoring body (4) and an opposite terminal end (17) for implanting said anchoring arm (9) in the second bone (3), two of the anchoring arms (10, 11) being disposed on either side of the axis (Y-Y') so as to extend along a common midplane parallel to the axis (Y-Y') and spaced from the latter.

8. The implant (1) according to any one of claims 1 to 5, **characterized in that** the anchoring body (4) extends along an axis (X-X') and the deformable portion (5) extending in a direction opposite to the anchoring body (4) along an axis (Y-Y') secant to the axis (X-X'), such that the axis (Y-Y') is inclined relative to the axis (X-X') at an elevation angle (a) comprised between:
- 8 and 12 degrees, preferably about 10 degrees, or
- 15 and 19 degrees, preferably about 17 degrees.

9. The implant (1) according to the preceding claim, **characterized in that** the anchoring body (4) has an external surface connecting the base end (7) to the penetrating end (6), the external surface (8) being generally convergent in shape, for example conical, in the direction of the penetrating end (6).

10. The implant (1) according to any one of the preceding claims, **characterized in that** it comprises a cannula (23) passing through the anchoring body (4) so as to prolong said free space (E) to the penetrating end (6).

11. The implant (1) according to any one of the preceding claims, **characterized in that** the anchoring body (4) extending along an axis (X-X') from the base end (7) to the penetrating end (6), said anchoring body (4) comprises at least one peripheral longitudinal furrow (24) allowing blocking the implant (1) in rotation about the axis (X-X') within the first bone (2), the longitudinal furrow (24) extending substantially parallel to the axis (X-X') from the penetrating end (6) over at least one portion of the length of the anchoring body (4).

12. The implant (1) according to the preceding claim, **characterized in that** it comprises four peripheral longitudinal furrows (24), such that the anchoring body (4) has a substantially cruciform orthogonal section from the penetrating end (6) over at least one portion of its length.

13. The implant (1) according to any one of the preceding claims, **characterized in that** it forms a phalangeal intra-medullary implant (1), the first bone (2) forming a first phalange and the second bone (3) forming a second phalange of the same finger as the first phalange, the anchoring body (4) being intended to be anchored in a medullary canal of the first bone (2), the deformable portion (5) being intended to be anchored in the medullary canal of the second bone (3).
